(19) Europäisches Patentamt
European Patent Office
Office européen des brevets

(11) **EP 4 079 222 A1**

(12) **EUROPEAN PATENT APPLICATION**
published in accordance with Art. 153(4) EPC

(43) Date of publication:
26.10.2022 Bulletin 2022/43

(51) International Patent Classification (IPC):
**A61B 5/145** (2006.01)

(21) Application number: 21812532.6

(22) Date of filing: 25.05.2021

(86) International application number:
PCT/CN2021/095859

(87) International publication number:
WO 2021/238934 (02.12.2021 Gazette 2021/48)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 29.05.2020 CN 202010476854

(71) Applicant: **Anhui Huami Health Technology Co., Ltd.**
**Hefei, Anhui 243000 (CN)**

(72) Inventors:
• **ZHU, Guokang**
**Hefei, Anhui 243000 (CN)**
• **WANG, Kongqiao**
**Hefei, Anhui 243000 (CN)**

(74) Representative: **Papa, Elisabetta et al**
**Società Italiana Brevetti S.p.A**
**Piazza di Pietra, 39**
**00186 Roma (IT)**

(54) **BLOOD OXYGEN SATURATION MEASUREMENT METHOD AND APPARATUS, STORAGE MEDIUM, AND ELECTRONIC DEVICE**

(57) Disclosed are a blood oxygen saturation measurement method and apparatus. Said method comprises: acquiring a pressure measurement signal between a blood oxygen saturation measurement apparatus and a part to be tested, so as to obtain a pressure measurement value; identifying that the pressure measurement value is within a preset range, and acquiring a blood oxygen saturation measurement signal of said part; and according to the blood oxygen saturation measurement signal of said part, obtaining a blood oxygen saturation measurement value of said part. Thus, a pressure measure-

ment value between the blood oxygen saturation measurement apparatus and a part to be tested is acquired, blood oxygen saturation information is not acquired temporarily when the blood oxygen saturation measurement apparatus is worn too loosely or too tightly, and blood oxygen saturation information is acquired when a pressure measurement value is within a preset range, avoiding the acquisition of erroneous blood oxygen saturation information, improving the accuracy of measurement by the blood oxygen saturation measurement apparatus.

obtaining a pressure measurement signal between a blood oxygen saturation measurement apparatus and a part to be measured, so as to obtain a pressure measurement value

in response to determining that the pressure measurement value is within a preset range, obtaining a blood oxygen saturation measurement signal of the part to be measured

obtaining a blood oxygen saturation measurement value of the part to be measured based on the blood oxygen saturation measurement signal of the part to be measured

FIG. 2

# EP 4 079 222 A1

**Description**

## CROSS-REFERENCE TO RELATED APPLICATIONS

This application claims priority to Chinese Patent Application No. 202010476854.2, titled "Blood Oxygen Saturation Measurement Method and Apparatus, Storage Medium and Electronic Device", filed on May 29, 2020 by Anhui Huami Health Technology Co., Ltd..

## TECHNICAL FIELD

The disclosure relates to the field of medical instruments, in particular to a method and an apparatus for measuring a blood oxygen saturation, a storage medium and an electronic device.

## BACKGROUND

With the development of smart wearable device technology, it is possible to use wearable devices to perform non-invasive physiological parameter measurements. For example, non-invasive blood oxygen saturation measurement can be performed to diagnose the sleep-disordered breathing syndrome, which is widely admired by users for convenience and is of great significance in clinical practice. The blood oxygen saturation is another important vital sign index other than pulse rate, body temperature, blood pressure and respiration. The clinical significance of the non-invasive blood oxygen saturation measurement has been widely recognized.

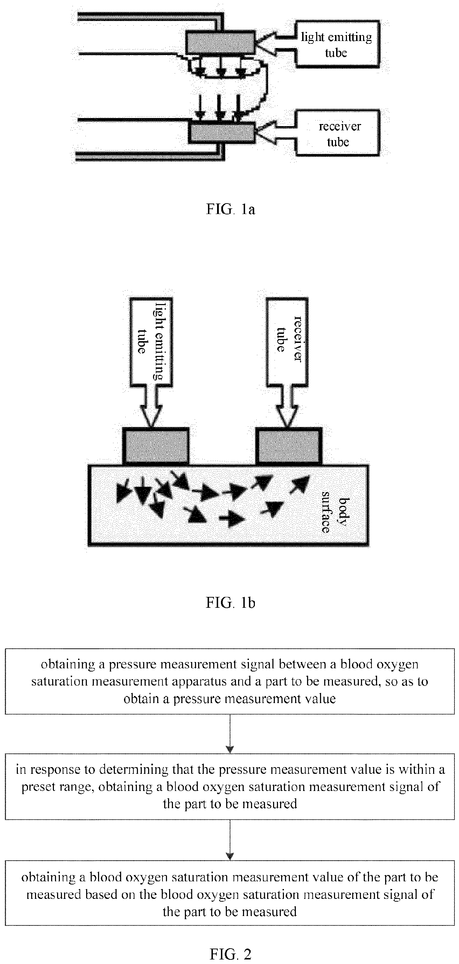

The non-invasive blood oxygen saturation measurement is mainly based on the Lambert-Beer law, to deduce the blood oxygen saturation by measuring absorption amounts of blood for lights of two different wavelengths according to Photo PlethysmoGraphy (PPG) technology based on the principle of different spectral absorption rates of oxyhemoglobin and deoxyhemoglobin. Generally, two LEDs of different wavelengths are used as light sources, and phototransistors or other photosensitive devices are used to collect signals. There are mainly two methods of transmission method and reflection method. As illustrated in FIG. 1a, the main structure difference between the transmission oximeter and the reflection oximeter is different relative positions of the light source and the sensor. The sensor and the light source of the transmission oximeter may be placed on different sides of the human tissue, and the light emitted from the light source travels through the tissue to the sensor. As illustrated in FIG. 1b, the sensor and the light source of the reflection oximeter are arranged on the same side of the tissue. After the light enters the tissue, it is reflected, scattered and then returned to be collected by the sensor.

In the related art, when the transmission oximeter measures the blood oxygen saturation, it must be ensured that the light can penetrate through the human tissue and generally act on fingers, toes, earlobes and nose. However, measurements on the above spots tend to limit the daily activities of users, which may cause discomfort. The reflection oximeter has less restrictions on the measurement spots, and can be worn on the wrist, arm, leg, forehead and most parts of the body, which has less interference on daily activities and has better portability.

However, the disclosure has come to the conclusion in the research process that the contact pressure between the device and the body tissue during the reflection measurement has a great influence on the measurement result. When the sensor is loosely attached to the body tissue (for example, there is a gap between the sensor and the body tissue), or the sensor is tightly attached to the body tissue and the body tissue is squeezed, the light signal cannot reliably reflect the blood oxygen saturation signs, and the existing devices cannot cope with these conditions and cannot guarantee correct measurement of the blood oxygen saturation.

## SUMMARY

The purpose of embodiments of the disclosure is to provide a method for measuring a blood oxygen saturation, which obtains a pressure measurement value between the blood oxygen saturation measurement apparatus and a part to be measured, determines whether the pressure measurement value is in a preset range, does not collect blood oxygen saturation information temporarily when a pressure between the blood oxygen saturation measurement apparatus and the part to be measured is too large or too small, and prompts the user to intervene in the measurement state, and collects the blood oxygen saturation information when the pressure measurement value is within the preset range, which avoids the acquisition of erroneous blood oxygen saturation information, and improves the accuracy of measurement by the blood oxygen saturation measurement apparatus.

In order to solve the above technical problem, according to a first aspect of the disclosure, a method for measuring a blood oxygen saturation is provided. The method includes: obtaining a pressure measurement signal between a blood oxygen saturation measurement apparatus and a part to be measured, so as to obtain a pressure measurement value; in response to determining that the pressure measurement value is within a preset range, obtaining a blood oxygen saturation measurement signal of the part to be measured; and obtaining a blood oxygen saturation measurement value of the part to be measured based on the blood oxygen saturation measurement signal of the part to be measured.

According to an embodiment of the disclosure, the method further includes: in response to determining that the pressure measurement value is not within the preset range, adjusting a pressure between the blood oxygen saturation measurement apparatus and the part to be measured.

According to an embodiment of the disclosure, in response to determining that the pressure measurement value is not within the preset range, adjusting the pressure between the blood oxygen saturation measurement apparatus and the part to be measured, includes: in response to determining that the pressure measurement value is less than a lower limit of the preset range, increasing the pressure between the blood oxygen saturation measurement apparatus and the part to be measured; or in response to determining that the pressure measurement value is greater than an upper limit of the preset range, reducing the pressure between the blood oxygen saturation measurement apparatus and the part to be measured.

According to an embodiment of the disclosure, the method further includes: obtaining a first error threshold and a second error threshold of the blood oxygen saturation measurement, in which the first error threshold is less than the second error threshold; obtaining a blood oxygen saturation measurement standard value; and determining the preset range based on a difference value between the blood oxygen saturation measurement value and the measurement standard value, the first error threshold and the second error threshold.

According to an embodiment of the disclosure, the preset range includes: a first preset range, a second preset range and a third preset range. An upper limit of the first preset range is equal to a lower limit of the second preset range, and an upper limit of the second preset range is equal to a lower limit of the third preset range.

According to an embodiment of the disclosure, determining the first preset range includes: in response to adjusting a pressure value between the blood oxygen saturation measurement apparatus and the part to be measured, an absolute value of the difference value between the blood oxygen saturation measurement value and the measurement standard value being the second error threshold, and the pressure value being inversely correlated with the absolute value, determining the pressure value as a lower limit of the first preset range; or in response to adjusting a pressure value between the blood oxygen saturation measurement apparatus and the part to be measured, an absolute value of the difference value between the blood oxygen saturation measurement value and the measurement standard value being the first error threshold, and the pressure value being inversely correlated with the absolute value, determining the pressure value as the upper limit of the first preset range.

According to an embodiment of the disclosure, determining the third preset range includes: in response to adjusting the pressure value between the blood oxygen saturation measurement apparatus and the part to be measured, the absolute value of the difference value between the blood oxygen saturation measurement value and the measurement standard value being the first error threshold, and the pressure value being positively correlated with the absolute value, determining the pressure value as the lower limit of the third preset range; or in response to adjusting the pressure value between the blood oxygen saturation measurement apparatus and the part to be measured, the absolute value of the difference value between the blood oxygen saturation measurement value and the measurement standard value being the second error threshold, and the pressure value being positively correlated with the absolute value, determining the pressure value as an upper limit of the third preset range.

According to an embodiment of the disclosure, before obtaining the blood oxygen saturation measurement value of the part to be measured, the method further includes: performing signal processing on the blood oxygen saturation measurement signal, in which the signal processing includes at least one of frequency domain signal processing, time domain signal processing and space domain signal processing.

According to an embodiment of the disclosure, performing the signal processing on the blood oxygen saturation measurement signal includes: obtaining a first blood oxygen saturation measurement signal with a frequency less than a frequency threshold by performing low-pass filtering on the blood oxygen saturation measurement signal; determining a second blood oxygen saturation measurement signal with a time domain stability parameter less than a stability threshold from the first blood oxygen saturation measurement signal; and determining a third blood oxygen saturation measurement signal with a signal peak amplitude parameter less than an amplitude threshold from the second blood oxygen saturation measurement signal.

According to an embodiment of the disclosure, the method further includes: in response to the pressure measurement value being in the second preset range, performing a first type of signal processing on the blood oxygen saturation measurement value; or in response to the pressure measurement value being in the first preset range or the third preset range, performing a second type of signal processing on the blood oxygen saturation measurement value, wherein a frequency threshold of the first type of signal processing is greater than a frequency threshold of the second type of signal processing, a stability threshold of the first type of signal processing is greater than a stability threshold of the second type of signal processing, and an amplitude threshold of the first type of signal processing is greater than an amplitude threshold of the second type of signal processing.

According to a second aspect of the disclosure, an apparatus for measuring a blood oxygen saturation is provided. The apparatus includes: a pressure measurement module, configured to obtain a pressure measurement signal between a blood oxygen saturation measurement apparatus and a part to be measured, so as to obtain a pressure measurement value; a blood oxygen saturation measurement module, configured to obtain a blood oxygen saturation measurement signal of the part to be measured in response to determining that the pressure measurement value is within a preset range, and to obtain a blood oxygen saturation measurement value of the part to be measured based on the blood oxygen saturation measurement signal of the part to be measured.

According to an embodiment of the disclosure, the apparatus further includes: an information prompt module, configured to, in response to determining that the pressure measurement value is not within the preset range, issue an information prompt signal, in which the information prompt signal is configured to instruct to adjust a pressure between the blood oxygen saturation measurement apparatus and the part to be measured.

According to an embodiment of the disclosure, the information prompt module includes a first information prompt unit and a second information prompt unit. The first information prompt unit is configured to, in response to determining that the pressure measurement value is less than a lower limit of the preset range, issue a first information prompt signal, in which the first information prompt signal is configured to instruct to increase the pressure between the blood oxygen saturation measurement apparatus and the part to be measured. The second information prompt unit is configured to, in response to determining that the pressure measurement value is greater than an upper limit of the preset range, issue a second information prompt signal, in which the second information prompt signal is configured to instruct to reduce the pressure between the blood oxygen saturation measurement apparatus and the part to be measured.

According to an embodiment of the disclosure, the apparatus further includes a preset range determining module. The preset range determining module is configured to: obtain a first error threshold and a second error threshold of the blood oxygen saturation measurement, in which the first error threshold is less than the second error threshold; obtain a blood oxygen saturation measurement standard value; and determine the preset range based on a difference value between the blood oxygen saturation measurement value and the measurement standard value, the first error threshold and the second error threshold.

According to an embodiment of the disclosure, the preset range includes: a first preset range, a second preset range and a third preset range. An upper limit of the first preset range is equal to a lower limit of the second preset range, an upper limit of the second preset range is equal to a lower limit of the third preset range.

The preset range determining module includes: a first determining unit, configured to, in response to adjusting a pressure value between the blood oxygen saturation measurement apparatus and the part to be measured, an absolute value of the difference value between the blood oxygen saturation measurement value and the measurement standard value being the second error threshold, and the pressure value being inversely correlated with the absolute value, determine the pressure value as a lower limit of the first preset range; a second determining unit, configured to, in response to adjusting a pressure value between the blood oxygen saturation measurement apparatus and the part to be measured, an absolute value of the difference value between the blood oxygen saturation measurement value and the measurement standard value being the first error threshold, and the pressure value being inversely correlated with the absolute value, determine the pressure value as the upper limit of the first preset range.

According to an embodiment of the disclosure, the preset range determining module includes: a third determining unit, configured to, in response to adjusting the pressure value between the blood oxygen saturation measurement apparatus and the part to be measured, the absolute value of the difference value between the blood oxygen saturation measurement value and the measurement standard value being the first error threshold, and the pressure value being positively correlated with the absolute value, determine the pressure value as the lower limit of the third preset range; a fourth determining unit, configured to, in response to adjusting the pressure value between the blood oxygen saturation measurement apparatus and the part to be measured, the absolute value of the difference value between the blood oxygen saturation measurement value and the measurement standard value being the second error threshold, and the pressure value being positively correlated with the absolute value, determine the pressure value as an upper limit of the third preset range.

According to an embodiment of the disclosure, the apparatus further includes: a determining module, configured to determine whether the pressure measurement value is within the preset range, and in response to the pressure measurement value being within the preset range, instruct the blood oxygen saturation measurement module to obtain the blood oxygen saturation measurement signal.

According to an embodiment of the disclosure, the blood oxygen saturation measurement module is further configured to: perform signal processing on the blood oxygen saturation measurement signal, in which the signal processing includes at least one of frequency domain signal processing, time domain signal processing and space domain signal processing.

According to an embodiment of the disclosure, the blood oxygen saturation measurement module further includes: a frequency domain signal processing unit, configured to obtain a first blood oxygen saturation measurement signal with a frequency less than a frequency threshold by performing low-pass filtering on the blood oxygen saturation measurement signal; a time domain signal processing unit, configured to determine a second blood oxygen saturation measurement signal with a time domain stability parameter less than a stability threshold from the first blood oxygen saturation measurement signal; a space domain signal processing unit, configured to determine a third blood oxygen saturation measurement signal with a signal peak amplitude parameter less than an amplitude threshold from the second blood oxygen saturation measurement signal; and a signal conversion unit, configured to obtain the blood oxygen saturation measurement value based on the third blood oxygen saturation measurement signal.

According to an embodiment of the disclosure, the pressure measurement module is configured to, in response to the pressure measurement value being in the second preset range, instruct the blood oxygen saturation measurement module to perform a first type of signal processing on the blood oxygen saturation measurement value. The pressure measurement module is configured to, in response to the pressure measurement value being in the first preset range or the third preset range, instruct the blood oxygen saturation measurement module to perform a second type of signal processing on the blood oxygen saturation measurement value.

The blood oxygen saturation measurement module is configured such that, a frequency threshold of the first type of signal processing is greater than a frequency threshold of the second type of signal processing, a stability threshold of the first type of signal processing is greater than a stability threshold of the second type of signal processing, and an amplitude threshold of the first type of signal processing is greater than an amplitude threshold of the second type of signal processing.

According to a third aspect of the disclosure, a computer storage medium having computer programs stored thereon is provided. When the computer programs are executed by a processor, the above blood oxygen saturation measurement method is implemented.

According to a fourth aspect of the disclosure, an electronic device is provided. The electronic device includes a memory, a processor, and computer programs stored on the memory and capable of running on the processor. When the computer programs are executed by a processor, the above blood oxygen saturation measurement method is implemented.

The above technical solution of embodiments of the disclosure has the following beneficial technical effects.

According to the method and apparatus for measuring a blood oxygen saturation of the embodiments of the disclosure, the pressure measurement value between the blood oxygen saturation measurement apparatus and the part to be measured is obtained, and the pressure value between the blood oxygen saturation measurement apparatus and the part to be measured is adjusted when the pressure measurement value is not in the preset range, and the blood oxygen saturation information is acquired when the pressure measurement value is within the preset range, which avoids the acquisition of erroneous blood oxygen saturation information, and improves the accuracy of measurement by the blood oxygen saturation measurement apparatus.

BRIEF DESCRIPTION OF THE DRAWINGS

FIG. 1a is a schematic diagram of principle of a transmission oximeter according to an embodiment of the disclosure.
FIG. 1b is a schematic diagram of principle of a reflection oximeter according to an embodiment of the disclosure.
FIG. 2 is a flowchart of a method for measuring a blood oxygen saturation according to an embodiment of the disclosure.
FIG. 3 is a schematic diagram of modules of an apparatus for measuring a blood oxygen saturation according to an embodiment of the disclosure.
FIG. 4 is a schematic diagram of an information prompt module according to an embodiment of the disclosure.
FIG. 5 is a schematic diagram of a preset range determining module according to an embodiment of the disclosure.

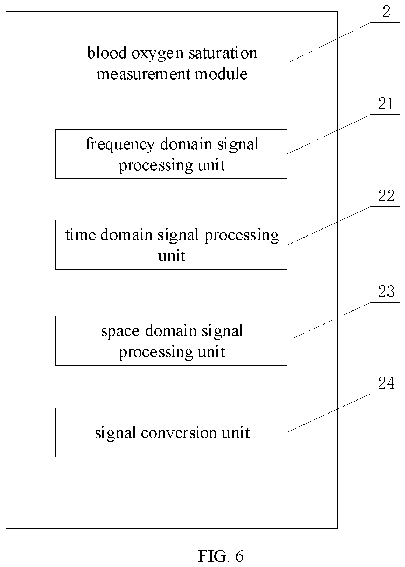

FIG. 6 is a schematic diagram of a blood oxygen saturation measurement module according to an embodiment of the disclosure.

Reference numbers:

pressure measurement module 1;
blood oxygen saturation measurement module 2;
frequency domain signal processing unit 21;
time domain signal processing unit 22;
space domain signal processing unit 23;
signal conversion unit 24;

information prompt module 3;
first information prompt unit 31;
second information prompt unit 32;
preset range determining module 4;
first determining unit 41;
second determining unit 42;
third determining unit 43;
fourth determining unit 44;
determining module 5

## DETAILED DESCRIPTION

In order to make the objectives, technical solutions and advantages of the disclosure clearer, the disclosure will be further described in detail below with reference to the specific embodiments and the accompanying drawings. It should be understood that these descriptions are exemplary only and are not intended to limit the scope of the disclosure. In addition, descriptions of well-known structures and techniques are omitted to avoid unnecessarily obscuring the concepts of the disclosure.

FIG. 2 is a flowchart of a method for measuring a blood oxygen saturation according to an embodiment of the disclosure.

As illustrated in FIG. 2, the first aspect of the embodiments of the disclosure provides a method for measuring a blood oxygen saturation. The method includes: obtaining a pressure measurement signal between a blood oxygen saturation measurement apparatus and a part to be measured, so as to obtain a pressure measurement value; in response to determining that the pressure measurement value is within a preset range, obtaining a blood oxygen saturation measurement signal of the part to be measured; and obtaining a blood oxygen saturation measurement value of the part to be measured based on the blood oxygen saturation measurement signal of the part to be measured.

Optionally, the pressure measurement value between the blood oxygen saturation measurement apparatus and the part to be measured may be obtained through a pressure measurement module of the blood oxygen saturation measurement apparatus, or may be obtained in other ways, which is not limited in the disclosure, as long as the acquisition of the pressure value between the blood oxygen saturation measurement apparatus and the part to be measured can be achieved, belongs to the protection scope of the disclosure.

According to an implementation of the disclosure, in response to determining that the pressure measurement value is not within the preset range, the pressure between the blood oxygen saturation measurement apparatus and the part to be measured is adjusted. For example, in response to determining that the pressure measurement value is less than a lower limit of the preset range, the pressure between the blood oxygen saturation measurement apparatus and the part to be measured is increased; or in response to determining that the pressure measurement value is greater than an upper limit of the preset range, the pressure between the blood oxygen saturation measurement apparatus and the part to be measured is reduced.

The preset range includes: a first preset range, a second preset range and a third preset range. An upper limit of the first preset range is equal to a lower limit of the second preset range, and an upper limit of the second preset range is equal to a lower limit of the third preset range. The first preset range and the third preset range are measurable ranges, and the second preset range is an ideal pressure range. In detail, the first preset range and the third preset range may be referred to as the measurable pressure ranges, and the second preset range may be referred to as the ideal pressure range. In addition, when the pressure measurement value is not in the preset range, it enters measurement rejection ranges.

Optionally, the meaning of the ideal pressure range is that if the pressure measurement value obtained by the blood oxygen saturation measurement apparatus is within the ideal pressure range, an error of the blood oxygen saturation obtained by further measurement is relatively small, which is less than a preset first error threshold. That is, when the pressure value measured by the blood oxygen saturation measurement apparatus is within this ideal pressure range, the measured blood oxygen saturation value is relatively accurate.

It can be understood that the meaning of the above measurable pressure range is that if the pressure measurement value measured by the blood oxygen saturation measurement apparatus is in the measurable pressure range, the error of the blood oxygen saturation measured by the blood oxygen saturation measurement apparatus is greater than the preset first error threshold but less than a preset second error threshold, and the error is within an acceptable range. That is, if the pressure measurement value obtained by the blood oxygen saturation measurement apparatus is within the measurable pressure range, the error can be reduced by processing the blood oxygen saturation signal obtained by measurement.

Optionally, the first preset range ranges from the first preset threshold to the second preset threshold. The second preset range ranges from the second preset threshold and the third preset threshold. The third preset range ranges from the third preset threshold to the fourth preset threshold.

According to an implementation of the disclosure, the method further includes: in response to determining that the pressure measurement value is not within the preset range, issuing a pressure adjustment signal, in which the pressure adjustment signal is configured to instruct to adjust the pressure between the blood oxygen saturation measurement apparatus and the part to be measured.

In detail, the pressure adjustment signal includes: a first pressure adjustment signal and a second pressure adjustment signal.

Optionally, in response to determining that the pressure measurement value is less than the first preset threshold, the first pressure adjustment signal is sent. The first pressure adjustment signal is configured to prompt that the blood oxygen saturation measurement apparatus is worn too loosely, and the blood oxygen saturation measurement apparatus needs to be attached tightly to the part to be measured.

Optionally, in response to determining that the pressure measurement value is greater than the fourth preset threshold, the second pressure adjustment signal is sent. The second pressure adjustment signal is configured to prompt that the blood oxygen saturation measurement apparatus is worn too tightly, and the blood oxygen saturation measurement apparatus needs to be loose to a certain extent to be attached to the part to be measured properly.

According to an implementation of the disclosure, the method further includes: in response to determining that the pressure measurement value is not within the preset range, adjusting the pressure between the blood oxygen saturation measurement apparatus and the part to be measured until the pressure measurement value is within the preset range.

According to an implementation of the disclosure, the step of determining the above preset range includes:

obtaining a first error threshold and a second error threshold of the blood oxygen saturation measurement, in which the first error threshold is less than the second error threshold, that is, the first error threshold is an upper limit of error for accurate measurement, and the second error threshold is an upper limit of error for acceptable measurement; and

obtaining a blood oxygen saturation measurement standard value, in which the blood oxygen saturation measurement standard value, for example, refers to that, when determining the above preset range, the user simultaneously uses a blood oxygen saturation measurement apparatus to be calibrated and a high-precision oximeter to measure the blood oxygen saturation value of testees in real time, and the blood oxygen saturation measurement value measured by the high-precision oximeter is the blood oxygen saturation measurement standard value.

Optionally, a pressure value S between the blood oxygen saturation measurement apparatus and the part to be measured is adjusted. For example, an absolute value E of the difference value between the blood oxygen saturation measurement value and the measurement standard value is a second error threshold $\tau 2$, or approximately the second error threshold $\tau 2$. The pressure value S is inversely correlated with the absolute value E, that is, when the pressure value S increases, the absolute value E decreases, and when the pressure value S decreases, the absolute value E increases. It is determined that the pressure value S is a lower limit S1 of the first preset range that can be used, which is the first preset threshold.

It can be understood that, the above method may also be used for multiple times to obtain multiple lower limits S1 of the first preset range, and an average value of the multiple lower limits S1 of the first preset range is set as S1.

Preferably, the absolute value E of the difference value between the blood oxygen saturation measurement value and the measurement standard value is about a second error threshold $\tau 2$, which means $\tau 2 \pm \tau 2 * 10\%$. Preferably, the second error threshold $\tau 2$ is 4%, where "%" is a concentration unit of blood oxygen saturation.

Optionally, the pressure value S between the blood oxygen saturation measurement apparatus and the part to be measured is adjusted. For example, the absolute value E of the difference value between the blood oxygen saturation measurement value S and the measurement standard value is the first error threshold $\tau 1$ or approximately the first error threshold $\tau 1$. The pressure value S is inversely correlated with the absolute value E, that is, when the pressure value S increases, the absolute value E decreases, and when the pressure value S decreases, the absolute value E increases. It is determined that the pressure value is an upper limit S2 of the first preset range, which is the second preset threshold.

The approximate first error threshold $\tau 1$ may refer to $\tau 1 \pm \tau 1 * 10\%$. Preferably, the first error threshold $\tau 1$ is 2%.

It can be understood that, the above method may also be used for multiple times to obtain multiple upper limits S2 of the first preset range, and an average value of the multiple upper limits S2 of the first preset range is set as S2.

Optionally, the pressure value S between the blood oxygen saturation measurement apparatus and the part to be measured is adjusted. For example, the absolute value E of the difference value between the blood oxygen saturation measurement value and the measurement standard value is the first error threshold or approximately the first error threshold. The pressure value S is positively correlated with the absolute value E, that is, the absolute value E increases when the pressure value S increases, and the absolute value E decreases when the pressure value S decreases. It is determined that the pressure value S is a lower limit S3 of the third preset range, which is the third preset threshold.

It can be understood that, the above method can also be used for multiple times to obtain multiple lower limits S3 of the third preset range, and an average value of the multiple lower limits S3 of the third preset range is set as S3.

Optionally, the pressure value S between the blood oxygen saturation measurement apparatus and the part to be measured is adjusted. For example, the absolute value of the difference value between the blood oxygen saturation measurement value and the measurement standard value is the second error threshold or approximately the second error threshold. The pressure value S is positively correlated with the absolute value E, that is, when the pressure value S increases, the absolute value E increases, and when the pressure value S decreases, the absolute value E decreases. The pressure value S is an upper limit S4 of the third preset range, that is, the fourth preset threshold.

It can be understood that, the above method can also be used for multiple times to obtain the upper limits S4 of the third preset range, and the average value of the upper limits S4 of the third preset range is set as S4.

Optionally, the PPG perfusion index can also be used to replace the first preset threshold, the second preset threshold, the third preset threshold and the fourth preset threshold. Through statistical analysis of a large number of experimental data, the range of values for the perfusion index that can reliably measure the blood oxygen saturation can be obtained. The above solution does not need to rely on the pressure sensor as the basis for adjustment, which reduces the hardware cost of the blood oxygen saturation measurement apparatus.

In addition, obtaining the blood oxygen saturation measurement standard value includes: obtaining the blood oxygen saturation measurement standard value by at least one high-precision blood oxygen saturation measurement apparatus. Optionally, to obtain the blood oxygen saturation measurement standard value, the blood oxygen saturation measurement value of the part to be measured can be obtained separately through multiple high-precision measurement apparatus, and then an average value of the obtained measurement values can be obtained and determined as the measurement standard value. The blood oxygen saturation measurement apparatus is calibrated to satisfy the need for higher accuracy for the individual user. Meanwhile, a large number of users can also be tested, and test values that are commonly used can be selected as the first preset threshold, the second preset threshold, the third preset threshold and/or the fourth preset threshold to meet the needs of ordinary users.

In the steps of the method for measuring a blood oxygen saturation, before obtaining the blood oxygen saturation measurement value of the part to be measured, the method further includes: performing signal processing on the blood oxygen saturation measurement signal, in which the signal processing includes at least one of frequency domain signal processing, time domain signal processing and space domain signal processing.

Optionally, performing the signal processing on the blood oxygen saturation measurement signal may include: obtaining a first blood oxygen saturation measurement signal with a frequency less than a frequency threshold by performing low-pass filtering on the blood oxygen saturation measurement signal; determining a second blood oxygen saturation measurement signal with a time domain stability parameter less than a stability threshold from the first blood oxygen saturation measurement signal; and determining a third blood oxygen saturation measurement signal with a signal peak amplitude parameter less than an amplitude threshold from the second blood oxygen saturation measurement signal.

In detail, the frequency domain signal processing includes: performing low-pass filtering on the blood oxygen saturation measurement signal to obtain the blood oxygen saturation measurement signal, in which a cut-off frequency $\theta f$ of the low-pass filtering processing is a first preset frequency.

In detail, the time domain signal processing includes: obtaining a time domain stability parameter of the blood oxygen saturation measurement signal, and obtaining the blood oxygen saturation measurement signal with a time domain stability parameter less than a first stability threshold. Optionally, the blood oxygen saturation measurement apparatus includes two optical signals, and the stability of the optical signal can be calculated through a sliding window, and the corresponding threshold $\theta t$ can be selected. The two optical signals in the sliding window are R and N respectively,

$$a = \sum (ri - \mu R)(ni - \mu N) / \sum (ni - \mu N)2,$$

$$E = MSE(a(N - \mu N) - (R - \mu R)) / MSE(R - \mu R),$$

where $ri \in R$, $ni \in N$, $\mu N$ and $\mu R$ are mean values of N and R respectively, and MSE () is a mean square error function. If $E > \theta t$, the signal in the sliding window is considered to be a bad signal and is not used to calculate the blood oxygen saturation.

Optionally, the space domain signal processing includes: obtaining signal peaks of the blood oxygen saturation measurement signal, and obtaining the blood oxygen saturation measurement value with a signal peak amplitude parameter less than a first amplitude threshold $\theta s$. Firstly, the signal peaks of the blood oxygen saturation measurement signal are extracted, and reference median filter result of peak amplitudes is selected based on the first amplitude threshold θs. The peak numbers are set as i=1,2,...m, and its corresponding amplitude is pi:

$$E = |pi - \text{median}(pj|\ i-1 \leqslant j \leqslant i+1)|,$$

where median() is a median function. If E>θs, a signal segment corresponding to pi is considered to be a bad signal, and is not used to calculate the blood oxygen saturation.

If the pressure measurement value is in the ideal measurement range, the signal processing rules are relaxed to perform the blood oxygen saturation calculation, that is, by increasing θf, θt and θs.

Optionally, after the blood oxygen saturation signal is processed through the above steps, the processed blood oxygen saturation signal is converted into the blood oxygen saturation measurement value. The existing algorithm may be used to convert the processed blood oxygen saturation signal into the blood oxygen saturation measurement value, which will not be repeated in the disclosure.

In an embodiment, when the pressure measurement value is in the second preset range, that is, the ideal measurement range, the first type of signal processing is performed on the blood oxygen saturation measurement value, in which the frequency threshold used in the first type of signal processing is the first frequency threshold, the stability threshold used in the first type of signal processing is the first stability threshold, and the amplitude threshold used in the first type of signal processing is the first amplitude threshold.

When the pressure measurement value is in the first preset range or the third preset range that is, a measurable range, the second type of signal processing is performed on the blood oxygen saturation measurement value, in which the frequency threshold used in the second type of signal processing is the second frequency threshold, the stability threshold is the second stability threshold, and the amplitude threshold is the second amplitude threshold.

The first frequency threshold is greater than the second frequency threshold, the first stability threshold is greater than the second stability threshold, and the first amplitude threshold is greater than the second amplitude threshold.

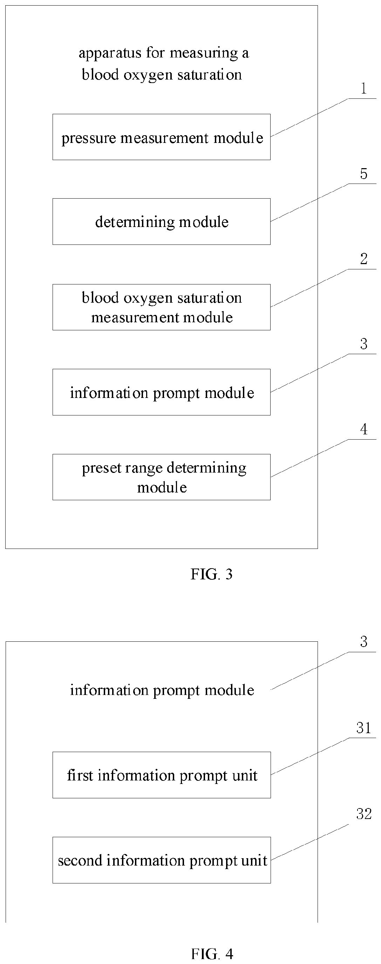

FIG. 3 is a schematic diagram of modules of an apparatus for measuring a blood oxygen saturation according to an embodiment of the disclosure.

As illustrated in FIG. 3, the second aspect of embodiments of the disclosure provides an apparatus for measuring a blood oxygen saturation. The apparatus includes: a pressure measurement module 1 and a blood oxygen saturation measurement module 2. The pressure measurement module 1 is configured to obtain a pressure measurement signal between a blood oxygen saturation measurement apparatus and a part to be measured, so as to obtain a pressure measurement value. The blood oxygen saturation measurement module 2 is configured to obtain a blood oxygen saturation measurement signal of the part to be measured in response to determining that the pressure measurement value is within a preset range, and to obtain a blood oxygen saturation measurement value of the part to be measured based on the blood oxygen saturation measurement signal of the part to be measured.

According to an implementation of the disclosure, the apparatus further includes: an information prompt module 3, configured to, in response to determining that the pressure measurement value is not within the preset range, issue an information prompt signal, in which the information prompt signal is configured to instruct to adjust a pressure between the blood oxygen saturation measurement apparatus and the part to be measured.

According to an implementation of the disclosure, the apparatus further includes: a pressure adjustment module, configured to, in response to determining that the pressure measurement value is not within the preset range, adjust a pressure between the blood oxygen saturation measurement apparatus and the part to be measured.

FIG. 4 is a schematic diagram of an information prompt module according to an embodiment of the disclosure.

As illustrated in FIG. 4, the information prompt module 3 includes a first information prompt unit 31 and a second information prompt unit 32.

The first information prompt unit 31 is configured to, in response to determining that the pressure measurement value is less than a lower limit of the preset range, issue a first information prompt signal, in which the first information prompt signal is configured to instruct to increase the pressure between the blood oxygen saturation measurement apparatus and the part to be measured.

The second information prompt unit 32 is configured to, in response to determining that the pressure measurement value is greater than an upper limit of the preset range, issue a second information prompt signal, in which the second information prompt signal is configured to instruct to reduce the pressure between the blood oxygen saturation measurement apparatus and the part to be measured.

According to an implementation of the disclosure, the apparatus further includes: a preset range determining module 4. The preset range determining module 4 is configured to: obtain a first error threshold and a second error threshold of the blood oxygen saturation measurement, in which the first error threshold is less than the second error threshold; obtain a blood oxygen saturation measurement standard value; and determine the preset range based on a difference value between the blood oxygen saturation measurement value and the blood oxygen saturation measurement standard value, the first error threshold and the second error threshold.

According to an implementation of the disclosure, the preset range includes: a first preset range, a second preset range and a third preset range. An upper limit of the first preset range is equal to a lower limit of the second preset range, an upper limit of the second preset range is equal to a lower limit of the third preset range.

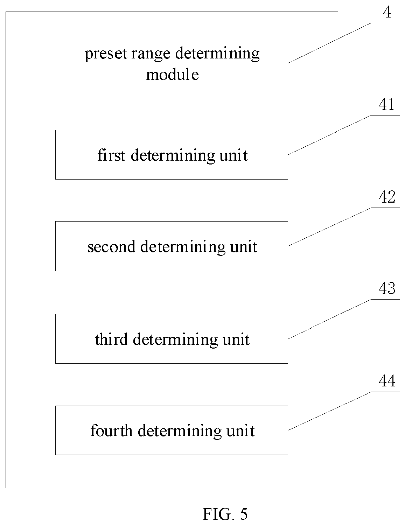

FIG. 5 is a schematic diagram of a preset range determining module according to an embodiment of the disclosure.

As illustrated in FIG. 5, the preset range determining module 4 includes: a first determining unit 41 and a second determining unit 42.

The first determining unit 41 is configured to, in response to adjusting a pressure value between the blood oxygen saturation measurement apparatus and the part to be measured, an absolute value of the difference value between the blood oxygen saturation measurement value and the blood oxygen saturation measurement standard value being the second error threshold, and the pressure value being inversely correlated with the absolute value, determine the pressure value as a lower limit of the first preset range.

The second determining unit 42 is configured to, in response to adjusting a pressure value between the blood oxygen saturation measurement apparatus and the part to be measured, an absolute value of the difference value between the blood oxygen saturation measurement value and the blood oxygen saturation measurement standard value being the first error threshold, and the pressure value being inversely correlated with the absolute value, determine the pressure value as the upper limit of the first preset range.

According to an implementation of the disclosure, the preset range determining module 4 includes: a third determining unit 43 and a fourth determining unit 44.

The third determining unit 43 is configured to, in response to adjusting the pressure value between the blood oxygen saturation measurement apparatus and the part to be measured, the absolute value of the difference value between the blood oxygen saturation measurement value and the blood oxygen saturation measurement standard value being the first error threshold, and the pressure value being positively correlated with the absolute value, determine the pressure value as the lower limit of the third preset range.

The fourth determining unit 44 is configured to, in response to adjusting the pressure value between the blood oxygen saturation measurement apparatus and the part to be measured, the absolute value of the difference value between the blood oxygen saturation measurement value and the blood oxygen saturation measurement standard value being the second error threshold, and the pressure value being positively correlated with the absolute value, determine the pressure value as an upper limit of the third preset range.

The first error threshold is the upper limit of error for accurate measurement, the second error threshold is the upper limit of error for acceptable measurement, and the blood oxygen saturation measurement standard value is the blood oxygen saturation measurement value in an ideal state.

Optionally, the blood oxygen saturation measurement standard value is the standard value of blood oxygen saturation measured in an ideal state by at least one high-precision blood oxygen saturation measurement apparatus.

According to an implementation of the disclosure, the apparatus further includes: a determining module 5, configured to determine whether the pressure measurement value is within the preset range, and in response to the pressure measurement value being within the preset range, instruct the blood oxygen saturation measurement module 2 to obtain the blood oxygen saturation measurement signal.

According to an implementation of the disclosure, the blood oxygen saturation measurement module 2 is further configured to: perform signal processing on the blood oxygen saturation measurement signal, in which the signal processing includes at least one of frequency domain signal processing, time domain signal processing and space domain signal processing.

According to an embodiment of the disclosure, the blood oxygen saturation measurement module 2 includes: a frequency domain signal processing unit 21, a time domain signal processing unit 22, a space domain signal processing unit 23 and a signal conversion unit 24.

The frequency domain signal processing unit 21 is configured to obtain a first blood oxygen saturation measurement signal with a frequency less than a frequency threshold by performing low-pass filtering on the blood oxygen saturation measurement signal.

The time domain signal processing unit 22 is configured to determine a second blood oxygen saturation measurement signal with a time domain stability parameter less than a stability threshold from the first blood oxygen saturation measurement signal.

The space domain signal processing unit 23 is configured to determine a third blood oxygen saturation measurement signal with a signal peak amplitude parameter less than an amplitude threshold from the second blood oxygen saturation measurement signal.

The signal conversion unit 24 is configured to obtain the blood oxygen saturation measurement value based on the third blood oxygen saturation measurement signal.

According to an embodiment of the disclosure, the pressure measurement module 1 is configured to, in response to the pressure measurement value being in the second preset range, instruct the blood oxygen saturation measurement module 2 to perform a first type of signal processing on the blood oxygen saturation measurement value. The pressure measurement module 1 is configured to, in response to the pressure measurement value being in the first preset range or the third preset range, instruct the blood oxygen saturation measurement module 2 to perform a second type of signal processing on the blood oxygen saturation measurement value. The blood oxygen saturation measurement module 2 is configured such that a frequency threshold of the first type of signal processing is greater than a frequency threshold of the second type of signal processing, a stability threshold of the first type of signal processing is greater than a stability threshold of the second type of signal processing, and an amplitude threshold of the first type of signal processing is greater than an amplitude threshold of the second type of signal processing.

According to a third aspect of the disclosure, a computer storage medium having computer programs stored thereon is provided. When the computer programs are executed by a processor, the above blood oxygen saturation measurement method is implemented.

According to a fourth aspect of the disclosure, an electronic device is provided. The electronic device includes: a memory, a processor, and computer programs stored on the memory and capable of running on the processor. When the computer programs are executed by a processor, the above blood oxygen saturation measurement method is implemented.

The disclosure provides a blood oxygen saturation measurement method. The method includes: obtaining a pressure measurement signal between a blood oxygen saturation measurement apparatus and a part to be measured, so as to obtain a pressure measurement value; in response to determining that the pressure measurement value is within a preset range, obtaining a blood oxygen saturation measurement signal of the part to be measured; and obtaining a blood oxygen saturation measurement value of the part to be measured based on the blood oxygen saturation measurement signal of the part to be measured. The disclosure further provides a blood oxygen saturation measurement apparatus, a storage medium and an electronic device. The above technical solution has the following beneficial effects.

According to the method and apparatus for measuring a blood oxygen saturation of the embodiments of the disclosure, the pressure measurement value between the blood oxygen saturation measurement apparatus and the part to be measured is obtained, and the pressure value between the blood oxygen saturation measurement apparatus and the part to be measured is adjusted when the pressure measurement value is not in the preset range, and the blood oxygen saturation information is acquired when the pressure measurement value is within the preset range, which avoids the acquisition of erroneous blood oxygen saturation information, and improves the accuracy of measurement by the blood oxygen saturation measurement apparatus.

It should be understood that the above specific embodiments of the disclosure are only used to illustrate or explain the principle of the disclosure, but not to limit the disclosure. Therefore, any modifications, equivalent replacements and improvements made without departing from the spirit and scope of the disclosure should be included in the protection scope of the disclosure. Furthermore, the appended claims of the disclosure are intended to cover all changes and modifications that fall within the scope and boundaries of the appended claims, or the equivalents of such scope and boundaries.

**Claims**

1. A method for measuring a blood oxygen saturation, comprising:

   obtaining a pressure measurement signal between a blood oxygen saturation measurement apparatus and a part to be measured, so as to obtain a pressure measurement value;
   in response to determining that the pressure measurement value is within a preset range, obtaining a blood oxygen saturation measurement signal of the part to be measured; and
   obtaining a blood oxygen saturation measurement value of the part to be measured based on the blood oxygen saturation measurement signal of the part to be measured.

2. The method of claim 1, further comprising:
   in response to determining that the pressure measurement value is not within the preset range, adjusting a pressure between the blood oxygen saturation measurement apparatus and the part to be measured.

3. The method of claim 2, wherein in response to determining that the pressure measurement value is not within the preset range, adjusting the pressure between the blood oxygen saturation measurement apparatus and the part to be measured, comprises:

in response to determining that the pressure measurement value is less than a lower limit of the preset range, increasing the pressure between the blood oxygen saturation measurement apparatus and the part to be measured; or

in response to determining that the pressure measurement value is greater than an upper limit of the preset range, reducing the pressure between the blood oxygen saturation measurement apparatus and the part to be measured.

4. The method of any one of claims 1-3, further comprising:

obtaining a first error threshold and a second error threshold of the blood oxygen saturation measurement, wherein the first error threshold is less than the second error threshold;
obtaining a blood oxygen saturation measurement standard value; and
determining the preset range based on a difference value between the blood oxygen saturation measurement value and the measurement standard value, the first error threshold and the second error threshold.

5. The method of claim 4, wherein the preset range comprises: a first preset range, a second preset range and a third preset range, an upper limit of the first preset range is equal to a lower limit of the second preset range, and an upper limit of the second preset range is equal to a lower limit of the third preset range.

6. The method of claim 5, wherein determining the first preset range comprises:

in response to adjusting a pressure value between the blood oxygen saturation measurement apparatus and the part to be measured, an absolute value of the difference value between the blood oxygen saturation measurement value and the measurement standard value being the second error threshold, and the pressure value being inversely correlated with the absolute value, determining the pressure value as a lower limit of the first preset range; or
in response to adjusting a pressure value between the blood oxygen saturation measurement apparatus and the part to be measured, an absolute value of the difference value between the blood oxygen saturation measurement value and the measurement standard value being the first error threshold, and the pressure value being inversely correlated with the absolute value, determining the pressure value as the upper limit of the first preset range.

7. The method of claim 5 or 6, wherein determining the third preset range comprises:

in response to adjusting the pressure value between the blood oxygen saturation measurement apparatus and the part to be measured, the absolute value of the difference value between the blood oxygen saturation measurement value and the measurement standard value being the first error threshold, and the pressure value being positively correlated with the absolute value, determining the pressure value as the lower limit of the third preset range; or
in response to adjusting the pressure value between the blood oxygen saturation measurement apparatus and the part to be measured, the absolute value of the difference value between the blood oxygen saturation measurement value and the measurement standard value being the second error threshold, and the pressure value being positively correlated with the absolute value, determining the pressure value as an upper limit of the third preset range.

8. The method of any one of claims 1-3 or 5, wherein before obtaining the blood oxygen saturation measurement value of the part to be measured, the method further comprises:
performing signal processing on the blood oxygen saturation measurement signal, wherein the signal processing comprises at least one of frequency domain signal processing, time domain signal processing and space domain signal processing.

9. The method of claim 8, wherein performing the signal processing on the blood oxygen saturation measurement signal comprises:

obtaining a first blood oxygen saturation measurement signal with a frequency less than a frequency threshold by performing low-pass filtering on the blood oxygen saturation measurement signal;
determining a second blood oxygen saturation measurement signal with a time domain stability parameter less than a stability threshold from the first blood oxygen saturation measurement signal; and

determining a third blood oxygen saturation measurement signal with a signal peak amplitude parameter less than an amplitude threshold from the second blood oxygen saturation measurement signal.

10. The method of claim 9, further comprising:

in response to the pressure measurement value being in the second preset range, performing a first type of signal processing on the blood oxygen saturation measurement value; or

in response to the pressure measurement value being in the first preset range or the third preset range, performing a second type of signal processing on the blood oxygen saturation measurement value; wherein

a frequency threshold of the first type of signal processing is greater than a frequency threshold of the second type of signal processing, a stability threshold of the first type of signal processing is greater than a stability threshold of the second type of signal processing, and an amplitude threshold of the first type of signal processing is greater than an amplitude threshold of the second type of signal processing.

11. An apparatus for measuring a blood oxygen saturation, comprising:

a pressure measurement module (1), configured to obtain a pressure measurement signal between a blood oxygen saturation measurement apparatus and a part to be measured, so as to obtain a pressure measurement value;

a blood oxygen saturation measurement module (2), configured to obtain a blood oxygen saturation measurement signal of the part to be measured in response to determining that the pressure measurement value is within a preset range, and to obtain a blood oxygen saturation measurement value of the part to be measured based on the blood oxygen saturation measurement signal of the part to be measured.

12. The apparatus of claim 11, further comprising:
an information prompt module (3), configured to, in response to determining that the pressure measurement value is not within the preset range, issue an information prompt signal, wherein the information prompt signal is configured to instruct to adjust a pressure between the blood oxygen saturation measurement apparatus and the part to be measured.

13. The apparatus of claim 12, wherein the information prompt module (3) comprises a first information prompt unit (31) and a second information prompt unit (32);

the first information prompt unit (31) is configured to, in response to determining that the pressure measurement value is less than a lower limit of the preset range, issue a first information prompt signal, wherein the first information prompt signal is configured to instruct to increase the pressure between the blood oxygen saturation measurement apparatus and the part to be measured; and

the second information prompt unit (32) is configured to, in response to determining that the pressure measurement value is greater than an upper limit of the preset range, issue a second information prompt signal, wherein the second information prompt signal is configured to instruct to reduce the pressure between the blood oxygen saturation measurement apparatus and the part to be measured.

14. The apparatus of any one of claims 11-13, wherein the apparatus comprises a preset range determining module (4), and the preset range determining module (4) is configured to:

obtain a first error threshold and a second error threshold of the blood oxygen saturation measurement, wherein the first error threshold is less than the second error threshold;

obtain a blood oxygen saturation measurement standard value; and

determine the preset range based on a difference value between the blood oxygen saturation measurement value and the measurement standard value, the first error threshold and the second error threshold.

15. The apparatus of claim 14, wherein the preset range comprises: a first preset range, a second preset range and a third preset range, an upper limit of the first preset range is equal to a lower limit of the second preset range, an upper limit of the second preset range is equal to a lower limit of the third preset range, and the preset range determining module (4) comprises:

a first determining unit (41), configured to, in response to adjusting a pressure value between the blood oxygen saturation measurement apparatus and the part to be measured, an absolute value of the difference value between the blood oxygen saturation measurement value and the measurement standard value being the second error threshold, and the pressure value being inversely correlated with the absolute value, determine the pressure value as a lower limit of the first preset range; and

a second determining unit (42), configured to, in response to adjusting a pressure value between the blood oxygen saturation measurement apparatus and the part to be measured, an absolute value of the difference value between the blood oxygen saturation measurement value and the measurement standard value being the first error threshold, and the pressure value being inversely correlated with the absolute value, determine the pressure value as the upper limit of the first preset range.

16. The apparatus of claim 15, wherein the preset range determining module (4) further comprises:

a third determining unit (43), configured to, in response to adjusting the pressure value between the blood oxygen saturation measurement apparatus and the part to be measured, the absolute value of the difference value between the blood oxygen saturation measurement value and the measurement standard value being the first error threshold, and the pressure value being positively correlated with the absolute value, determine the pressure value as the lower limit of the third preset range; and

a fourth determining unit (44), configured to, in response to adjusting the pressure value between the blood oxygen saturation measurement apparatus and the part to be measured, the absolute value of the difference value between the blood oxygen saturation measurement value and the measurement standard value being the second error threshold, and the pressure value being positively correlated with the absolute value, determine the pressure value as an upper limit of the third preset range.

17. The apparatus of claim 11, further comprising:
a determining module (5), configured to determine whether the pressure measurement value is within the preset range, and in response to the pressure measurement value being within the preset range, instruct the blood oxygen saturation measurement module (2) to obtain the blood oxygen saturation measurement signal.

18. The apparatus of claim 11, wherein the blood oxygen saturation measurement module (2) is further configured to: perform signal processing on the blood oxygen saturation measurement signal, wherein the signal processing comprises at least one of frequency domain signal processing, time domain signal processing and space domain signal processing.

19. The apparatus of claim 18, wherein the blood oxygen saturation measurement module (2) further comprises:

a frequency domain signal processing unit (21), configured to obtain a first blood oxygen saturation measurement signal with a frequency less than a frequency threshold by performing low-pass filtering on the blood oxygen saturation measurement signal;

a time domain signal processing unit (22), configured to determine a second blood oxygen saturation measurement signal with a time domain stability parameter less than a stability threshold from the first blood oxygen saturation measurement signal; and

a space domain signal processing unit (23), configured to determine a third blood oxygen saturation measurement signal with a signal peak amplitude parameter less than an amplitude threshold from the second blood oxygen saturation measurement signal; and

a signal conversion unit (24), configured to obtain the blood oxygen saturation measurement value based on the third blood oxygen saturation measurement signal.

20. The apparatus of claim 19, wherein,

the pressure measurement module (1) is configured to, in response to the pressure measurement value being in the second preset range, instruct the blood oxygen saturation measurement module (2) to perform a first type of signal processing on the blood oxygen saturation measurement value;

the pressure measurement module (1) is configured to, in response to the pressure measurement value being in the first preset range or the third preset range, instruct the blood oxygen saturation measurement module (2) to perform a second type of signal processing on the blood oxygen saturation measurement value; wherein a frequency threshold of the first type of signal processing is greater than a frequency threshold of the second type of signal processing, a stability threshold of the first type of signal processing is greater than a stability threshold of the second type of signal processing, and an amplitude threshold of the first type of signal processing is greater than an amplitude threshold of the second type of signal processing.

21. A computer storage medium having computer programs stored thereon, wherein when the computer programs are executed by a processor, the method for measuring a blood oxygen saturation according to any one of claims 1-10 is implemented.

22. An electronic device, comprising a memory, a processor, and computer programs stored on the memory and capable of running on the processor, wherein when the computer programs are executed by a processor, the method for measuring a blood oxygen saturation according to any one of claims 1-10 is implemented.

FIG. 1a

FIG. 1b

obtaining a pressure measurement signal between a blood oxygen
saturation measurement apparatus and a part to be measured, so as to
obtain a pressure measurement value

in response to determining that the pressure measurement value is within a
preset range, obtaining a blood oxygen saturation measurement signal of
the part to be measured

obtaining a blood oxygen saturation measurement value of the part to be
measured based on the blood oxygen saturation measurement signal of
the part to be measured

FIG. 2

apparatus for measuring a
blood oxygen saturation

1

pressure measurement module

5

determining module

2

blood oxygen saturation
measurement module

3

information prompt module

4

preset range determining
module

FIG. 3

3

information prompt module

31

first information prompt unit

32

second information prompt unit

FIG. 4

preset range determining module ⟋ 4

first determining unit ⟋ 41

second determining unit ⟋ 42

third determining unit ⟋ 43

fourth determining unit ⟋ 44

FIG. 5

2

blood oxygen saturation
measurement module

21

frequency domain signal
processing unit

22

time domain signal processing
unit

23

space domain signal
processing unit

24

signal conversion unit

FIG. 6

## INTERNATIONAL SEARCH REPORT

| International application No. |
| --- |
| **PCT/CN2021/095859** |

**A. CLASSIFICATION OF SUBJECT MATTER**

A61B 5/145(2006.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNABS; CNTXT; CNKI; VEN; USTXT; EPTXT; WOTXT: 安徽华米, 朱国康, 汪孔桥, 血氧, 氧饱和, 压力, 区间, 阈值, 门限, 预设, 预定, 上限, 下限, 范围, 误差, 理想, 准确, 精确, 最优, 最佳, 正相关, 反相关, 增, 减, 大, 小, saturation, oximet+, pressure, force, interval, threshold+, predefin+, preset, predetermin+, limit, range, error?, ideal, accurate, precise, optimal, best, positive correlat+, negative correlat+, increas+, decreas+

**C. DOCUMENTS CONSIDERED TO BE RELEVANT**

| Category* | Citation of document, with indication, where appropriate, of the relevant passages | Relevant to claim No. |
| --- | --- | --- |
| PX | CN 111588385 A (ANHUI HUAMI INTELLIGENT TECHNOLOGY CO., LTD.) 28 August 2020 (2020-08-28) <br> claims 1-21, description paragraphs [0042]-[0114], figures 1a-6 | 1-22 |
| X | CN 103228205 A (NITTO DENKO CORPORATION) 31 July 2013 (2013-07-31) <br> description paragraphs [0130]-[0134], [0157]-[0172], [0191]-[0193], figures 5-23 | 1-3, 11-13, 17, 21, 22 |
| Y | CN 103228205 A (NITTO DENKO CORPORATION) 31 July 2013 (2013-07-31) <br> description paragraphs [0130]-[0134], [0157]-[0172], [0191]-[0193], figures 5-23 | 4-10, 14-16, 18-22 |
| Y | US 2016361016 A1 (TVERSKOY BORIS) 15 December 2016 (2016-12-15) <br> description, paragraphs [0052]-[0056], figure 1 | 4-10, 14-16, 18-22 |
| X | WO 2020008380 A1 (MIR SRL MEDICAL INTERNATIONAL RES) 09 January 2020 (2020-01-09) <br> description page 8 line 5- page 13 line 19, figures 1-4 | 1-3, 11-13, 17, 21, 22 |
| Y | WO 2020008380 A1 (MIR SRL MEDICAL INTERNATIONAL RES) 09 January 2020 (2020-01-09) <br> description page 8 line 5- page 13 line 19, figures 1-4 | 4-10, 14-16, 18-22 |

☑ Further documents are listed in the continuation of Box C.  ☑ See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **12 July 2021** | **02 August 2021** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088** <br> **China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2021/095859** |

**C.    DOCUMENTS CONSIDERED TO BE RELEVANT**

| Category* | Citation of document, with indication, where appropriate, of the relevant passages | Relevant to claim No. |
| --- | --- | --- |
| X | WO 2009086452 A1 (NELLCOR PURITAN BENNETT LLC et al.) 09 July 2009 (2009-07-09)<br>        description page 28 line 10- page 29 line 19, figure 12 | 1-3, 11-13, 17, 21, 22 |
| Y | WO 2009086452 A1 (NELLCOR PURITAN BENNETT LLC et al.) 09 July 2009 (2009-07-09)<br>        description page 28 line 10- page 29 line 19, figure 12 | 4-10, 14-16, 18-22 |
| A | CN 101884541 A (BEIHANG UNIVERSITY) 17 November 2010 (2010-11-17)<br>        entire document | 1-22 |

Form PCT/ISA/210 (second sheet) (January 2015)

**INTERNATIONAL SEARCH REPORT**
Information on patent family members

International application No.

**PCT/CN2021/095859**

| Patent document cited in search report | | | Publication date (day/month/year) | Patent family member(s) | | | Publication date (day/month/year) |
|---|---|---|---|---|---|---|---|
| CN | 111588385 | A | 28 August 2020 | None | | | |
| CN | 103228205 | A | 31 July 2013 | EP | 2632326 | A4 | 27 August 2014 |
| | | | | SG | 189431 | A1 | 31 May 2013 |
| | | | | AU | 2012207635 | A1 | 02 May 2013 |
| | | | | JP | 5771288 | B2 | 26 August 2015 |
| | | | | CA | 2815643 | A1 | 26 July 2012 |
| | | | | WO | 2012099535 | A1 | 26 July 2012 |
| | | | | CN | 103228205 | B | 30 September 2015 |
| | | | | JP | 2014507209 | A | 27 March 2014 |
| | | | | AU | 2012207635 | B2 | 16 July 2015 |
| | | | | CA | 2815643 | C | 15 January 2019 |
| | | | | MY | 171217 | A | 02 October 2019 |
| | | | | SG | 189431 | B | 31 July 2013 |
| | | | | EP | 2632326 | A1 | 04 September 2013 |
| US | 2016361016 | A1 | 15 December 2016 | US | 10342486 | B2 | 09 July 2019 |
| WO | 2020008380 | A1 | 09 January 2020 | None | | | |
| WO | 2009086452 | A1 | 09 July 2009 | None | | | |
| CN | 101884541 | A | 17 November 2010 | CN | 101884541 | B | 16 January 2013 |

Form PCT/ISA/210 (patent family annex) (January 2015)

**EP 4 079 222 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010476854